Europäisches Patentamt

European Patent Office

Office européen des brevets

(11) Numéro de publication: **0 209 468**
**A1**

## (12) DEMANDE DE BREVET EUROPEEN

(21) Numéro de dépôt: 86401628.2

(22) Date de dépôt: 21.07.86

(51) Int. Cl.4: **A61C 17/02 , A61B 17/22**

(30) Priorité: 19.07.85 FR 8511106

(43) Date de publication de la demande:
**21.01.87 Bulletin 87/04**

(84) Etats contractants désignés:
**AT BE CH DE GB IT LI LU NL SE**

(71) Demandeur: **SATELEC Société à Responsabilité Limitée**
**Zone Industrielle du Phare B.P. No. 216**
**F-33708 Merignac Cédex(FR)**

(72) Inventeur: **Dieras, Francis**
**16 Cours Xavier Arnozan**
**F-33000 Boreaux(FR)**
Inventeur: **Soulie, Alain**
**La Garenne - Daulet Canejan**
**F-33610 Cestas(FR)**
Inventeur: **Escanecrabe, Bernard**
**9, Parc d'Ornon Pont de la Maye**
**F-33140 Villenave d'Ornon(FR)**
Inventeur: **Larrieu, Jean-Louis**
**Lotissement l'Orée du Bois Lot 44**
**F-38138 Lanton(FR)**

(74) Mandataire: **Bruder, Michel**
**10 rue de la Pépinière**
**F-75008 Paris(FR)**

(54) Appareil de curetage ou d'exérèse de tissus biologiques par un instrument vibrant à des fréquences ultrasonores.

(57) La présente invention concerne un appareil à ultrasons permettant le curetage ou l'exérèse des tissus biologiques par irrigation d'un liquide soumis à la cavitation et par aspiration du tissu désagrégé, comprenant une pièce à main contenant un transducteur lequel est accouplé mécaniquement à un instrument vibrant ou sonotrode.

Cet appareil est caractérisé en ce que la pièce à main (1) est traversée de part en part, dans le sens longitudinal, par un conduit d'aspiration (15,24) relié, d'une part, à un raccord d'aspiration (3) se trouvant à l'extrémité postérieure de la pièce à main (1), et, d'autre part, à la partie interne de la sonotrode d'aspiration (17)

Fig. 1

0 209 468

La présente invention concerne un appareil de curetage ou d'exérèse de tissus biologiques par un instrument vibrant à des fréquences ultrasonores, associé à un ou à des fluides irrigants mis en cavitation et absorbé(s) par un système d'aspiration coaxial.

Des appareils utilisant les ultrasons pour le traitement des surfaces ont été proposés depuis longtemps. C'est le cas dans l'industrie, comme dans le domaine médical, notamment en chirurgie dentaire. L'extrême rapidité des vibrations ultrasonores transmises à un instrument permettent de détacher et de réduire, en fines particules, des dépôts tels que le tartre.

Un tel appareil est d'autant plus efficace que l'on peut lui adjoindre une projection de fluide ou de poudre abrasive, venant éroder la partie à détacher qui peut être ensuite récupérée par un système d'aspiration.

Une telle combinaison de moyens à savoir projection de particules ou de fluide(s), ultrasons appliqués à un outil et aspiration, est d'ailleurs décrite dans le brevet US-2 874 470. Ce brevet fait état de l'intérêt de cette combinaison, pour son efficacité ainsi que pour ses avantages ergonomiques.

La présente invention reprend la combinaison de ces moyens, irrigation et aspiration, en adoptant pour chacune de ces fonctions une configuration coaxiale telle que l'on peut la voir figurer dans le brevet US-2 709 852 qui décrit un appareil dentaire, ou dans le brevet 3 089 790, qui décrit un appareil de nettoyage à ultrasons, avec irrigation et aspiration coaxiales.

Il existe bien, dans le domaine chirurgical, des appareils reprenant de la même manière ce type de configuration et utilisant la même combinaison de moyens. Toutefois, ces appareils connus sous le nom d'aspirateurs chirurgicaux à ultrasons présentent un certain nombre d'inconvénients.

D'une part, la pièce à main n'intègre pas entièrement les circuits des fluides notamment le conduit d'aspiration et le conduit d'irrigation avec pour conséquence une augmentation exagérée du diamètre de cette pièce à main. Toute opération délicate est rendue ainsi difficile et fatigante pour l'opérateur. La magnétostriction utilisée dans les appareils connus ne permet pas de remédier à ces inconvénients.

D'autre part, la technique de l'aspiration chirurgicale à ultrasons connue a l'inconvénient d'arracher les tissus dans certains cas, en particulier dans le cas où l'aspiration est mal réglée. Inversement, dans d'autres cas, l'effet destructeur est insuffisant et le procédé présente des risques pour les tissus adjacents dans le cas d'exérèse de certaines tumeurs, kystiques notamment.

Dans un tel appareil connu, il n'y a pas d'autre solution, dans les cas difficiles réquérant une grande efficacité de l'appareil, que le renforcement de l'aspiration ou des ultrasons appliqués à l'instrument vibrant unique appelé encore "sonotrode". Dans ce dernier cas, la puissance peut être telle que l'échauffement soit important et que l'amplitude obtenue à l'extrémité de la sonotrode soit dommageable pour les tissus adjacents et les vaisseaux.

A cet effet cet appareil à ultrasons permettant le curetage ou l'exérèse des tissus biologiques par irrigation d'un liquide soumis à la cavitation et par aspiration du tissu désagrégé, comprenant une pièce à main contenant un transducteur lequel est accouplé mécaniquement à un instrument vibrant ou sonotrode, est caractérisé en ce que la pièce à main est traversée de part en part, dans le sens longitudinal, par un conduit d'aspiration relié, d'une part, à un raccord d'aspiration se trouvant à l'extrémité postérieure de la pièce à main, et, d'autre part, à la partie interne de la sonotrode d'aspiration.

L'appareil suivant l'invention a pour avantages :

-d'intégrer les circuits des fluides d'un bout à l'autre de la pièce à main en offrant la possibilité d'avoir un diamètre d'aspiration important;

-d'alléger au maximum cette pièce à main qui se présente comme un tube;

-d'avoir, grâce au haut rendement de la piézo-électricité, un échauffement minimal de l'ensemble. Grâce à une adaptation automatique de l'amplitude de la vibration de la pièce à main en fonction de la résistance de tissu rencontré, il est possible d'agir efficacement sur toute sorte de tissu.

Il est possible d'utiliser soit des rondelles en céramiques piézo-électriques polarisées suivant l'épaisseur, soit un tube en céramique piézo-électrique polarisé radialement, ce qui a pour avantage de libérer au maximum l'intérieur même du transducteur. Cet avantage permet de pouvoir combiner un ensemble de moyens, dont l'aspiration et l'irrigation, dans un volume restreint, mais aussi il permet d'intégrer coaxialement d'autres moyens tels une visée en fibre optique et/ou un faisceau laser.

L'utilisation de plusieurs sonotrodes fixées sur le même transducteur permet de moduler l'effet de coupe et d'éviter tout risque de déchirement tissulaire. L'effet de cavitation est important du fait de l'intégration du fluide d'irrigation dans une sonotrode.

On décrira ci-après, à titre d'exemples non limitatifs, diverses formes d'exécution de la présente invention, en référence au dessin annexé sur lequel :

La figure 1 est une vue en coupe axiale d'un appareil suivant l'invention.

Les figures 2a, 2b, 2c sont des vues en coupe axiale partielle de diverses formes d'exécution des parties extrêmes des sonotrodes.

La figure 3 est une vue en coupe axiale d'une variante d'exécution simplifiée d'une pièce à main.

La figure 4 est une vue en coupe transversale faite suivant la ligne IV-IV de la figure 3.

La figure 5 est une vue en élévation d'une sonotrode unique à écoulement longitudinal externe du fluide d'irrigation.

La figure 6 est une vue en élévation - schématique d'une variante d'exécution d'une sonotrode à écoulement externe hélicoïdal du fluide d'irrigation.

La figure 7 est une vue en élévation - schématique d'une autre variante d'exécution d'une sonotrode à écoulement longitudinal externe du fluide d'irrigation.

La figure 8 est une vue en coupe axiale partielle d'une variante d'exécution de l'appareil.

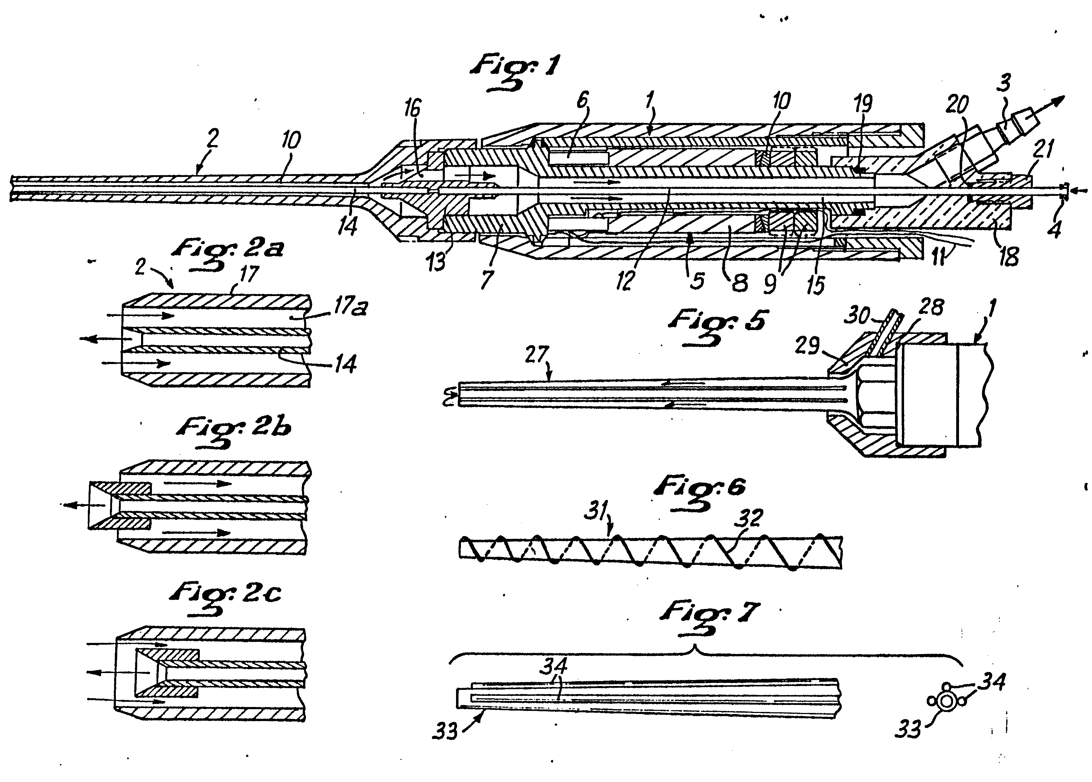

L'appareil suivant l'invention représenté sur la figure 1 comprend de la manière habituelle une pièce à main 1 contenant un générateur de vibrations ultrasonores ou transducteur 5 prolongé, à son extrémité avant, par un instrument allongé 2 soumis à des vibrations ultrasonores axiales et constitué d'une ou plusieurs sonotrodes coaxiales. A son extrémité arrière la pièce à main 1 porte des raccords à savoir un raccord 3 relié à une source de vide, afin de réaliser une aspiration, et un raccord 4 d'entrée d'un fluide d'irrigation.

Le transducteur 5 comprend un tube en céramique piézo-électrique 6 qui est maintenu bloqué entre une partie avant 7, de diamètre relativement grand, et une entretoise 8 bloquée au moyen d'écrous de serrage 9, la pression de serrage étant assurée par deux rondelles de précontrainte 10. Le tube piézo-électrique 6 est relié à l'extérieur par des fils électriques de connexion 11 qui sortent de l'extrémité arrière de la pièce à main 1. Comme on peut le voir sur la figure 1, la partie centrale du transducteur 5 est creuse et elle permet le passage de part en part, à travers la pièce à main 1, d'un fluide d'irrigation et du courant d'aspiration. Dans la forme d'exécution non limitative représentée sur la figure 1 le transducteur est traversé axialement de part en part par un tube axial d'irrigation 12 qui est relié, à son extrémité arrière, au raccord 4 d'entrée du fluide d'irrigation. Ce tube d'irrigation 12 est solidaire de la partie avant 7 du transducteur 5 de manière à être soumis aux vibrations ultrasonores, et ce par l'intermédiaire d'une pièce de raccordement 13. Cette pièce de raccordement 13 est percée de part en part d'un conduit central et elle est reliée, à sa partie antérieure, à un tube axial 14 s'étendant sur toute la longueur de l'instrument vibrant 2. Ce tube 14 constitue ainsi une sonotrode d'irrigation de l'appareil.

Par ailleurs le transducteur 5 est traversé de part en part, dans le sens axial, par au moins un conduit d'aspiration 15 qui est décalé vers l'extérieur par rapport au tube d'irrigation 12 et qui communique avec le raccord d'aspiration 3. Dans l'exemple illustré sur le dessin le conduit d'aspiration 15 est constitué par le volume interne du transducteur 5 qui entoure le tube d'irrigation central 12. Ce conduit d'aspiration 15 communique, par l'intermédiaire d'au moins un trou 16 percé de part en part dans la pièce de raccordement 13, avec au moins un conduit d'aspiration s'étendant longitudinalement à l'intérieur de la paroi de l'instrument vibrant 2 qui forme la sonotrode d'aspiration. Le conduit interne de la sonotrode d'aspiration 17 est en fait constitué, dans cette forme d'exécution, par le volume entourant le conduit central de la sonotrode d'irrigation 14.

Dans la partie postérieure de la pièce à main 1 est logé un bloc de raccordement 18 qui est relié à l'extrémité postérieure du transducteur 5, avec interposition d'un joint d'étanchéité torique 19 et qui est traversé axialement par la partie postérieure du tube d'irrigation 12 s'étendant jusqu'au raccord d'irrigation 4. Un autre joint d'étanchéité torique 20 est disposé dans le bloc de raccordement 8 autour du tube d'irrigation 12 et il est maintenu serré par un presse-étoupe 21 traversé par le tube d'irrigation 12 et vissé dans la partie postérieure du bloc de raccordement 18.

Les deux sonotrodes d'irrigation 14 et d'aspiration 17 peuvent se terminer, à leurs extrémités antérieures, soient dans un même plan transversal comme il est illustré sur la figure 2a, soit dans des plans transversaux différents. Dans le cas illustré sur la figure 2b la sonotrode d'irrigation interne 14 est en saillie par rapport à la sonotrode d'aspiration externe 17 tandis que la figure 2c montre la disposition inverse dans laquelle la sonotrode d'aspiration externe 17 s'étend au-delà de la sonotrode d'irrigation interne 14. Par ailleurs on peut voir sur les figures 2a, 2b et 2c que les parties extrêmes des sonotrodes se présentent sous forme biseautée, la partie extrême biseautée de la sonotrode d'aspiration externe 17 convergeant en direction de l'axe tandis que la partie extrême biseautée de la sonotrode d'irrigation interne 14 a une forme divergente.

Les positions différentes que peuvent occuper les sonotrodes l'une par rapport à l'autre, ainsi que leurs formes, ont des effets différents sur les tissus et ainsi sur l'efficacité de l'appareil.

Dans le cas illustré sur la figure 2a c'est-à-dire lorsque les extrémités des deux sonotrodes sont situées dans un même plan transversal, il est possible d'obtenir un effet de fragmentation localisée et en surface.

Dans le cas illustré sur la figure 2b, c'est-à-dire lorsque la sonotrode d'irrigation interne 14 dépasse de la sonotrode d'aspiration externe 17, les vibrations des deux sonotrodes 14 et 17 sont déphasées entre elles du fait de leurs diamètres différents ce qui a pour effet d'obtenir avec l'aspiration un phénomène de cisaillement des tissus.

Dans le cas illustré sur la figure 2c, c'est-à-dire lorsque la sonotrode d'irrigation 14 est située à l'intérieur même de la sonotrode d'aspiration 17, on obtient une véritable lyse tissulaire qui n'altère en rien la coupe sélective de l'instrument vibrant 2 à son extrémité.

Grâce à la configuration à deux sonotrodes 14 et 17 qui a été décrite ci-dessus, on obtient un effet conjugué de deux instruments à vibrations ultrasonores qui renforce la cavitation et évite, pour être très efficace d'avoir à augmenter la puissance du transducteur ou de l'aspiration. Pour pouvoir libérer la vision du champ opératoire il est possible de faire en sorte que l'axe de l'instrument vibrant 2 ne soit plus aligné avec l'axe de la pièce à main 1, comme il est représenté sur la figure 1, mais qu'au contraire il forme avec celui-ci un angle pouvant aller de 0 à 90° avec des pièces d'adaptation intermédiaire à raccorder. On peut également avoir recours à une rallonge intermédiaire droite entre la pièce à main 1 et l'instrument 2.

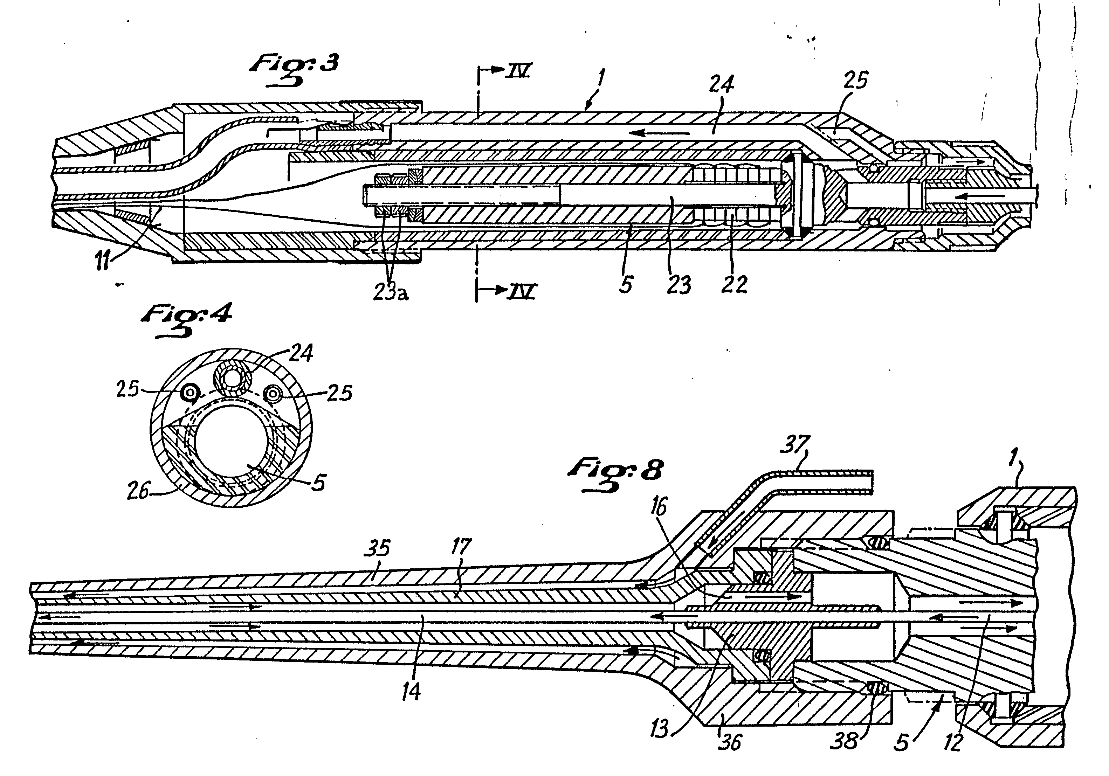

Les figures 3 et 4 montrent une variante simplifiée d'une pièce à main 1 obtenue grâce à l'utilisation de la piézo-électricité. Dans ce cas le transducteur 5 utilise, à la place d'un tube piézo-électrique, un ensemble de pastilles 22 montées en série et serrées les unes contre les autres aux moyens d'un tirant axial 23 et d'écrous 23a. Du fait que le transducteur 5 forme un ensemble compact et sans possibilité de passage à travers lui, le conduit d'irrigation 24 et le ou les conduits d'aspiration 25 sont prévus, toujours à l'intérieur de la pièce à main 11, mais à l'extérieur du transducteur 5. Plus particulièrement, comme on peut mieux le voir sur la figure 4, les conduits d'aspiration 25 et d'irrigation 24 sont logés dans l'espace disponible compris entre le transducteur 5 et la paroi cylindrique 26 de la pièce à main 1.

Dans la variante d'exécution illustrée sur la figure 5 l'appareil suivant l'invention comprend une sonotrode unique 27 d'aspiration et d'irrigation, qui est accouplée mécaniquement, comme dans les cas précédents, au tranducteur logé dans la pièce à main 1. Cette sonotrode unique 27 présente, sur sa surface latérale, une ou plusieurs rainures longitudinales qui permettent au liquide d'irrigation de cheminer, sous l'effet des ultrasons, jusqu'à l'extrémité de la sonotrode. A cet endroit le liquide est aspiré à l'intérieur de la sonotrode 27 avec les débris entraînés, et le courant d'aspiration s'écoule axialement à l'intérieur de la sonotrode 27 et à travers un conduit dans la pièce à main 1 jusqu'au raccord d'aspiration. L'alimentation en liquide d'irrigation s'effectue au moyen d'une pièce de raccordement annulaire 28 qui est fixée sur la partie extrême antérieure de la pièce à main 1 et qui entoure l'embase de la sonotrode 27, en laissant un passage libre 29 entre la pièce de raccordement 28 et la base de la sonotrode 27. Ce passage permet au liquide d'irrigation, qui est amené par un tube 30 débouchant à l'intérieur de la pièce de raccordement 28, de s'écouler sur les rainures longitudinales de la sonotrode 27 en direction de l'extrémité de celle-ci.

La figure 6 représente une variante d'exécution d'une sonotrode unique 31 d'aspiration et d'irrigation qui présente, sur sa surface latérale, au moins un filet hélicoïdal 32 canalisant l'écoulement du liquide d'irrigation qui, comme dans le cas précédent chemine, sous l'effet des ultrasons, en direction de l'extrémité de la sonotrode 31.

Dans la variante d'exécution illustrée sur la figure 7 la sonotrode unique d'aspiration et d'irrigation 33 comprend une tige creuse, de forme légèrement conique, dont le conduit axial central est relié, à travers la pièce à main, au raccord d'aspiration et qui porte, sur sa surface latérale, un ou plusieurs tubes longitudinaux d'irrigation 34.

Dans la variante d'exécution de l'invention représentée sur la figure 8 l'appareil qui a été illustré et décrit en référence à la figure 1, est pourvu d'une troisième sonotrode coaxiale 35 qui entoure la sonotrode d'aspiration 17, elle-même contenant la sonotrode d'irrigation 14. Cette troisième sonotrode 35 fait partie intégrante d'une embase 36 qui porte un tube 37 de raccordement à une source de fluide d'irrigation et qui est fixée, par tous moyens appropriés et avec interposition d'un joint d'étanchéité 38, sur la partie antérieure du transducteur 5. Un tel dispositif offre l'avantage de permettre l'acheminement de plusieurs liquides de traitement sur une zone à traiter et ce principe est particulièrement intéressant lorsqu'il est nécessaire de mettre ces liquides en présence au dernier moment.

Par ailleurs l'appareil suivant l'invention permet de transmettre également de part en part, à travers la pièce à main 1 et l'instrument vibrant 2, un faisceau de lumière chaude pour obtenir, par exemple, une hémostase immédiate. On peut utiliser une sonotrode bipolaire à effet diathermique ainsi qu'un faisceau laser, chacun de ces effets pouvant être combiné.

## Revendications

1.-Appareil à ultrasons permettant le curetage ou l'exérèse des tissus biologiques par irrigation d'un liquide soumis à la cavitation et par aspiration du tissu désagrégé, comprenant une pièce à main contenant un transducteur lequel est accouplé mécaniquement à un instrument vibrant ou sonotrode, caractérisé en ce que la pièce à main (1) est traversée de part en part, dans le sens longitudinal, par un conduit d'aspiration (15,24) relié, d'une part, à un raccord d'aspiration (3) se trouvant à l'extrémité postérieure de la pièce à main (1), et, d'autre part, à la partie interne de la sonotrode d'aspiration (17).

2.-Appareil suivant la revendication 1 caractérisé en ce que le ou les conduits d'irrigation (12,25) s'étendent longitudinalement de part en part au travers de la pièce à main (1).

3.-Appareil suivant la revendication 2 caractérisé en ce que le conduit d'irrigation (12) s'étend axialement de part en part à l'intérieur du transducteur (5).

4.-Appareil suivant la revendication 2 caractérisé en ce que le ou les conduits d'irrigation (25) s'étendent longitudinalement dans un espace compris entre le transducteur (5) et la paroi (26) de la pièce à main (1).

5.-Appareil suivant la revendication 1 caractérisé en ce que le ou les conduits d'irrigation s'étendent sur la surface d'une sonotrode unique d'aspiration et d'irrigation (27,31,33) et ils sont reliés à l'intérieur d'une pièce de raccordement (28) qui est fixée sur la partie antérieure de la pièce à main (1) et qui est reliée par un tube (30) à une source de fluide d'irrigation, la pièce de raccordement (28) délimitant avec l'embase de la sonotrode (27,31,33) un passage pour le fluide d'irrigation vers l'extrémité antérieure de la sonotrode.

6.-Appareil suivant la revendication 5 caractérisé en ce que la sonotrode (27) porte, sur sa surface latérale, des rainures longitudinales assurant le cheminemant du fluide d'irrigation.

7.-Appareil suivant la revendication 5 caractérisé en ce que la sonotrode (31) porte, sur sa surface latérale, au moins un filet hélicoïdal (32) guidant le cheminement du liquide d'irrigation.

8.-Appareil suivant la revendication 5 caractérisé en ce que la sonotrode (33) porte, sur sa surface latérale, au moins un tube longitudinal (34) assurant l'écoulement du fluide du liquide d'irrigation.

9.-Appareil suivant l'une quelconque des revendications précédentes caractérisé en ce qu'il comprend au moins deux sonotrodes coaxiales à savoir une sonotrode d'aspiration externe (17) et une sonotrode d'irrigation interne (14).

10.-Appareil suivant la revendication 9 caractérisé en ce qu'il comprend une troisième sonotrode d'irrigation externe (35) entourant la sonotrode d'aspiration (17), dont l'embase (36) est accouplée au transducteur (5) et porte un tube (37) d'alimentation en liquide d'irrigation.

11.-Appareil suivant l'une quelconque des revendications 9 et 10 caractérisé en ce que les extrémités des sonotrodes coaxiales (14,17,35) sont situées dans un même plan transversal ou dans des plans transversaux différents.

12.-Appareil suivant l'une quelconque des revendications précédentes caractérisé en ce que la pièce à main (1) et l'instrument vibrant (2) sont traversés de part en part, longitudinalement, par des moyens permettant la transmission, à travers eux, d'un faisceau de lumière chaude et/ou d'éclairage et/ou d'un laser de coupe, de traitement ou d'hémostase.

13.-Appareil suivant l'une quelconque des revendications précédentes caractérisé en ce qu'il comprend des moyens pour polariser la ou les sonotrodes de manière à donner un effet d'hémostase.

Fig.1

Fig.2a

Fig.2b

Fig.2c

Fig.5

Fig.6

Fig.7

0 209 468

Fig. 3

Fig. 4

Fig. 8

0 209 468

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | US-A-4 428 748 (PEYMAN)<br>* Figures 1,2; colonne 2, lignes 46-60; colonne 3, lignes 19-61; colonne 4, lignes 51-68; colonne 5, lignes 1-5 * | 1 | A 61 C 17/02<br>A 61 B 17/22 |
| Y | | 2,3 | |
| A | | 12 | |
| | --- | | |
| Y | US-A-3 518 766 (BURT)<br>* Figure 1; abrégé; colonne 4, lignes 72-75; colonne 5, lignes 1-17 * | 2,3 | |
| A | | 5,6 | DOMAINES TECHNIQUES RECHERCHES (Int. Cl.4) |
| | --- | | |
| X | US-A-3 941 122 (JONES)<br>* Figure; colonne 7, lignes 16-63 * | 1,2,4 | A 61 C<br>A 61 M<br>A 61 F<br>A 61 B |
| Y | | 5 | |
| | --- | | |
| Y | US-A-4 515 583 (SORICH)<br>* Figure 1; colonne 3, lignes 29-65 * | 5 | |
| | ---     -/- | | |

Le présent rapport de recherche a été établi pour toutes les revendications

| Lieu de la recherche | Date d'achèvement de la recherche | Examinateur |
|---|---|---|
| LA HAYE | 07-10-1986 | VANRUNXT J.M.A. |

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 316 465 (DOTSON)<br>* Figures 2,3; colonne 2, lignes 7-39; colonne 5, lignes 21-26 * | 1,2,4 | |
| | --- | | |
| A | US-A-3 636 947 (BALAMUTH)<br>* Figure 9; colonne 10, lignes 46-56 * | 5,8 | |
| | ----- | | |

DOMAINES TECHNIQUES RECHERCHES (Int. Cl.4)

Le present rapport de recherche a été établi pour toutes les revendications

| Lieu de la recherche | Date d'achèvement de la recherche | Examinateur |
|---|---|---|
| LA HAYE | 07-10-1986 | VANRUNXT J.M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82